(19) **Europäisches Patentamt**

**European Patent Office**

**Office européen des brevets**

(11) Veröffentlichungsnummer: **0 326 122 B1**

(12) # EUROPÄISCHE PATENTSCHRIFT

(45) Veröffentlichungstag der Patentschrift: **25.11.92**

(51) Int. Cl.⁵: **B65D 83/14**

(21) Anmeldenummer: **89101300.5**

(22) Anmeldetag: **26.01.89**

(54) Vorrichtung zur Erhöhung der Dosierungssicherheit von Aerosolpräparaten auf Suspensionsbasis.

(30) Priorität: **28.01.88 DE 3802498**

(43) Veröffentlichungstag der Anmeldung:
**02.08.89 Patentblatt 89/31**

(45) Bekanntmachung des Hinweises auf die
Patenterteilung:
**25.11.92 Patentblatt 92/48**

(84) Benannte Vertragsstaaten:
**AT BE CH DE ES FR GB GR IT LI LU NL SE**

(56) Entgegenhaltungen:
**DE-U- 8 713 851**
**FR-A- 2 615 172**

**AEROSOL AGE, Band 27, Nr. 8, August 1982, Seiten 36-40, New Jersey, US; R.E. SCHAR-MACH: "Dosage reproducibility of inhalation metering aerosol drug dose delivery systems"**

(73) Patentinhaber: **BOEHRINGER INGELHEIM KG**
**Postfach 200**
**W-6507 Ingelheim am Rhein(DE)**

(84) Benannte Vertragsstaaten:
**BE CH DE ES FR GR IT LI LU NL SE AT**

(73) Patentinhaber: **BOEHRINGER INGELHEIM INTERNATIONAL GmbH**
**Postfach 20**
**W-6507 Ingelheim am Rhein(DE)**

(84) Benannte Vertragsstaaten:
**GB**

(72) Erfinder: **Knecht, Adolf, Dr.**
**117er Ehrenhof 5**
**W-6500 Mainz(DE)**
Erfinder: **Daab, Ottfried, Dr.**
**Schillerstrasse 2a**
**W-6507 Ingelheim am Rhein(DE)**
Erfinder: **Weil, Hans-Hermann**
**Am Römer 2**
**W-6551 Gau-Bickelheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Vorrichtung zur Erhöhung der Dosierungssicherheit bei der therapeutischen Anwendung von Aerosolpräparaten auf Suspensionsbasis.

Suspensionen von feinstgemahlenen Arzneistoffen in der unter Druck flüssigen Phase der Treibmittel (z.B. Fluorchlorkohlenwasserstoffe) spielen vor allem in der Asthmatherapie eine große Rolle. Die Suspensionen werden in Druckbehälter mit Dosierventil abgefüllt. Mit Hilfe des Dosierventils ist es möglich, jeweils ein bestimmtes Volumen der Suspension zu dosieren, das durch spontanes Verdampfen des Treibmittels in ein inhalierfähiges Aerosol umgewandelt wird.

Aus therapeutischen Gründen ist es erforderlich, daß die bei jeder Betätigung des Dosierventils abgegebene Wirkstoffmenge möglichst wenig schwankt. Voraussetzung dafür ist die gleichmäßige Verteilung der Wirkstoffpartikel in der flüssigen Phase.

Wirkstoffe, deren Dichte größer ist als die der verflüssigten Treibmittel, setzen sich bei längerem Stehen ab. Befindet sich der Aerosolbehälter dabei in der Anwendungsposition (Ventil nach unten), so kommt es zu einer Erhöhung der Wirkstoffkonzentration in der Nähe der Einlaßöffnung des Dosierventils. Je nach den Umständen des Falls kann die Konzentrationserhöhung, die u.a. von dem Dichteunterschied Treibmittel/Arzneistoff und der Dauer der Ruhephase abhängt, beträchtlich sein. Eine homogene Suspension läßt sich im allgemeinen leicht durch Schütteln, Stoßen, Kippen und auch durch die Erschütterungen beim Transport und Mitsichführen rekonstituieren. Sofern indessen entgegen den Gebrauchshinweisen für solche Suspensionsdosieraerosole vor der Anwendung nicht geschüttelt wird und auch keine der anderen erwähnten Bewegungen erfolgen, kann eine stark erhöhte Wirkstoffdosis in das Dosierventil gelangen. Als Folge davon kann im ungünstigsten Fall die mit einem Aerosolstoß abgegebene Wirkstoffmenge ein Mehrfaches der beabsichtigten therapeutischen Dosis betragen. Hinweise auf die Dosierungsprobleme bei Suspensionsdosieraerosolen finden sich z.B. in AEROSOL AGE, Bd. 27, S. 36 - 40, New Jersey, USA; R.E. SCHARMACH; doch ist die volle Bedeutung dieser Probleme nicht zuletzt in Abhängigkeit von der Dichte des verwendeten verflüssigten Treibgases, offenbar bisher nicht erkannt worden.

Aufgabe der Erfindung ist es, die Gefahr der Überdosierung bei Suspensionsdosieraerosolen mit Wirkstoffen, die zur Sedimentation neigen, dadurch zu verhindern, daß durch bestimmte Vorrichtungen die Erhöhung der Wirkstoffkonzentration in der Nähe der Einlaßöffnung des Dosierventils weitestgehend vermieden wird.

Diese Aufgabe wird gemäß der Erfindung durch Konstruktionselemente gelöst, die jeweils für sich oder in beliebiger Kombination in die Dosieraerosolbehälter eingebaut werden.

Die Konstruktionselemente sind:

1. ein trichterförmiges Element, das das Ventil umschließt und nach oben (in Anwendungsposition des Behälters) geöffnet ist und nur einen Durchlaß, z.B. einen Ringspalt, in der Nähe der Behälterwandung offen läßt;

2. eine Abdeckplatte unterhalb des Trichters, die das Ventilgehäuse ringförmig umgibt und die lichte Weite des Behälters weitestgehend ausfüllt;

3. ein ringförmiges Element unterhalb der Abdeckplatte, das das Volumen im Bereich der Einlaßöffnung des Ventils weitestgehend verringert.

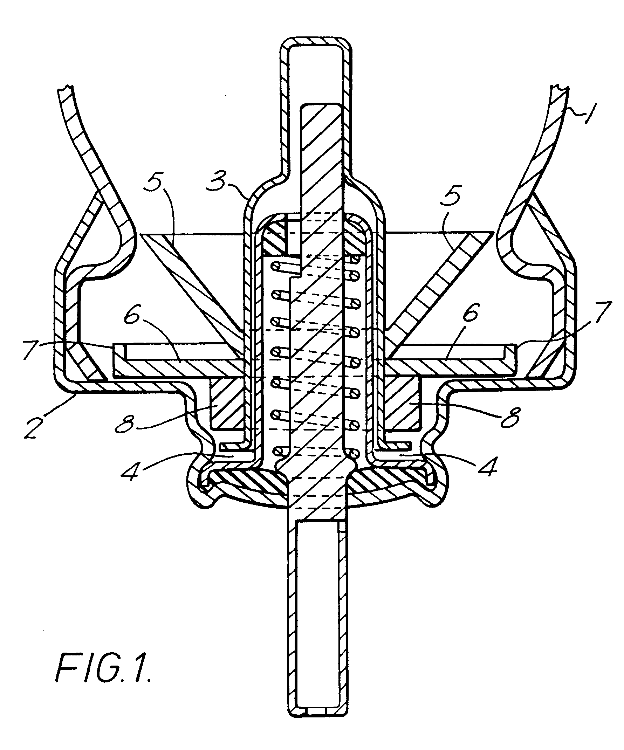

Figur 1 stellt eine bevorzugte Ausführungsform der Erfindung dar, die als Beispiel dienen soll. Es versteht sich, daß die Konstruktionselemente auch bei anderen üblichen Dosierventilen in nötigenfalls adaptierter Form verwendet werden können.

In Figur 1 bezeichnet 1 den Aerosolbehälter, 2 die aufgebördelte Verschlußkappe, an der das Ventil 3 befestigt ist. Das Ventil besitzt eine Einlaßöffnung 4 für die Suspension.

Die erfindungsgemäßen Konstruktionselemente sind ein trichterförmiges Teil 5, unterhalb dessen die Abdeckplatte 6 mit einem erhöhten Rand 7 angebracht ist, während das Volumen im verjüngten Teil der Verschlußkappe 2 durch das Element 8 teilweise ausgefüllt ist.

Zwischen den Elementen 5, 6 und 8 und der Wandung des Behälters bzw. der Verschlußkappe bleibt soviel Zwischenraum, daß die Funktion des Ventils nicht beeinträchtigt wird und die Suspension in ausreichendem Maß an diesen Elementen vorbei zu der Einlaßöffnung 4 gelangen kann.

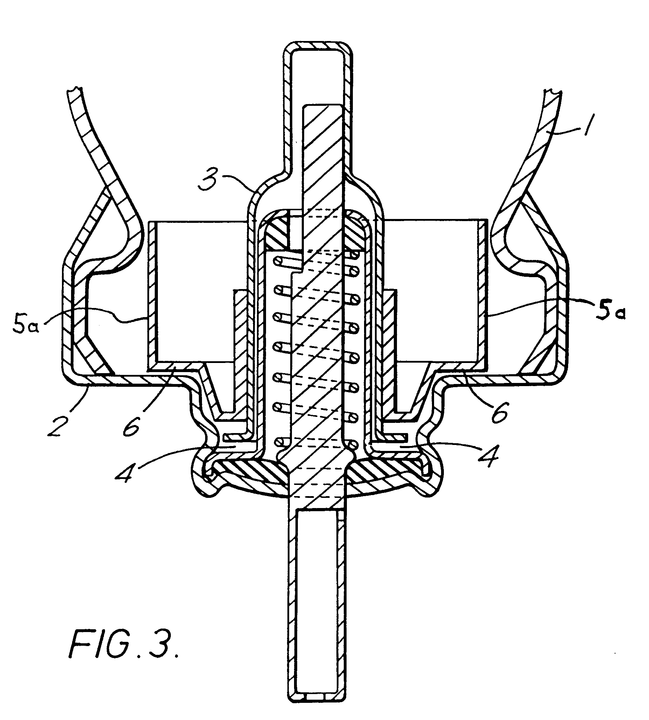

Bei dem in Figur 1 und 3 dargestellten Ventiltyp ist der Vortank (äußerer Ventilteil) am Ventilstamm befestigt und bewegt sich daher mit ihm. Im Fall von Ventilen, bei denen der Ventilkörper sich nicht bewegt, können die Konstruktionselemente so gestaltet sein, daß sie an der Wandung des Behälters anliegen und als Durchlass kleine Öffnungen (z.B. Kanäle, Rinnen) in der Nähe der Wandung aufweisen.

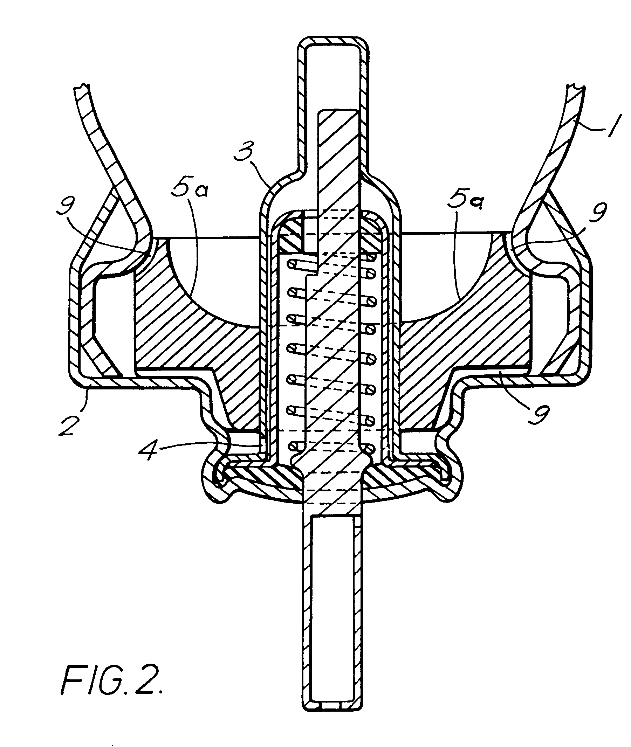

Eine solche Ausführungsform gibt Figur 2 wieder. Dort sind das trichterförmige Teil 5 und das volumenvermindernde Teil 8 unter Verzicht auf die Abdeckplatte 6 zu dem Element 5a kombiniert. Als Durchlaß für die Suspension sind Rinnen 9 vorgesehen, da das Teil 5 im oberen Bereich an der Wandung des Aerosolbehälters, im unteren Bereich an der Wandung der Verschlußkappe anliegt und das Ventilgehäuse dicht umschließt.

Eine weitere Ausführungsform der Erfindung ist in Figur 3 dargestellt, wo die Elemente 5 und 6 ebenfalls (auf etwas andere Weise) zu dem Element 5a zusammengefaßt sind.

Die Elemente 5, 6 und 8 können aus Materialien gefertigt werden, die gegenüber der Suspension inert sind, z.B. Kunststoff, Edelstahl, Aluminium. Die Elemente werden beispielsweise so gestaltet, daß sie in geeigneter Weise am Ventilgehäuse befestigt werden können oder auch einen Bestandteil desselben bilden.

**Patentansprüche**

1. Vorrichtung für die verbesserte Dosierung von Aerosolpräparaten auf Suspensionsbasis mit Wirkstoffen, die zur Sedimentation neigen, aus üblichen Aerosolbehältern mit Dosierventil, dadurch gekennzeichnet, daß um das Ventilgehäuse (3) in der Betätigungsposition oberhalb der Einlaßöffnung (4)
   a) ein nach oben geöffentes trichterförmiges Konstruktionselement (5),
   b) ein ringförmiges volumenminderndes Element (8) in der Nähe der Einlaßöffnung des Ventils und gegebenenfalls zwischen beiden
   c) eine ringförmige Abdeckplatte (6) mit nach oben gerichtetem Außenrand das Ventilgehäuse dicht umschließend
   angeordnet sind, wobei die unter a) bis c) genannten Elemente jeweils den Zwischenraum zwischen der Innenseite der Wand des Aerosolbehälters und dem Ventilgehäuse weitestgehend bis auf einen Durchlaß verschließen, der aus einem Ringspalt oder kleinen Öffnungen in der Nähe der Behälterwand besteht.

2. Vorrichtung nach Anspruch 1, dadurch gekennzeichnet, daß die Elemente gemäß 1a), 1b) und gegebenenfalls 1c) aus einem Stück bestehen.

3. Vorrichtung nach Anspruch 1 oder 2, dadurch gekennzeichnet, daß die Elemente gemäß 1a), 1b) und/oder 1c) mit den in den Behälter ragenden äußeren Ventilteilen (3) eine Einheit bilden.

4. Vorrichtung nach Anspruch 1, 2 oder 3, dadurch gekennzeichnet, daß das trichterförmige Element gemäß 1a) und das volumenvermindernde Element gemäß 1b) unter Verzicht auf die Abdeckplatte gemäß 1c) zu einem Element (5a) zusammengefaßt sind.

**Claims**

1. Apparatus for improved metering of suspension-based aerosol preparations containing active substances with a tendency to settle, from conventional aerosol containers with a metering valve, characterised in that about the valve housing (3) in the operating position above the inlet opening (4) are mounted
   a) a funnel shaped construction element (5) which is open at the top,
   b) an annular, volume-reducing element (8) in the region of the inlet opening of the valve, and possibly, between them,
   c) an annular cover plate (6) with a raised outer edge tightly enclosing the valve housing,
   whilst the elements mentioned in a) to c) substantially close off the space between the interior of the wall of the aerosol container and the valve housing, apart from an opening which consists of an annular slot or small openings near the container wall.

2. Apparatus as claimed in claim 1, characterised in that the elements according to 1a), 1b) and optionally 1c) are in one piece.

3. Apparatus as claimed in claim 1 or 2, characterised in that the elements according to 1a), 1b) and/or 1c) form a unit with the outer valve parts (3) projecting into the container.

4. Apparatus as claimed in claim 1, 2 or 3, characterised in that the funnel-shaped element according to 1a) and the volume reducing element according to 1b) are combined to form an element (5a), thus dispensing with the cover plate according to 1c).

**Revendications**

1. Dispositif pour augmenter la fiabilité du dosage de préparations en suspension pour aérosols contenant des principes actifs qui ont tendance à sédimenter, depuis des récipients courants pour aérosols, munis d'une valve doseuse, caractérisé en ce que
   a) un élément de construction (5) en forme d'entonnoir ouvert vers le haut,
   b) un élément annulaire (8) de réduction du volume à proximité de l'orifice d'entrée de la valve et, éventuellement, entre ces deux éléments
   c) une plaque de recouvrement (6) annulaire à rebord externe dirigé vers le haut, qui entoure étroitement le corps de la valve
   sont disposés autour du corps (3) de la valve en position d'actionnement, au dessus de l'orifice d'entrée (4), les éléments cités en a) à c) fermant largement l'interstice entre le côté interne de la paroi du récipient pour aérosol et le corps de la valve jusqu'à un passage qui consiste en une fente annulaire ou en de petits orifices à proximité de la paroi du récipient.

2. Dispositif selon la revendication 1, caractérisé en ce que les éléments selon 1a), 1b) et éventuellement 1c) sont d'une seule pièce.

3. Dispositif selon la revendication 1 ou 2, caractérisé en ce que les éléments selon 1a), 1b) et/ou 1c) forment une unité avec les parties externes (3) de la valve qui font saillie dans le récipient.

4. Dispositif selon la revendication 1, 2 ou 3, caractérisé en ce que l'élément en forme d'entonnoir selon 1a) et l'élément de réduction du volume selon 1b) sont combinés en un élément (5a) tandis que la plaque de recouvrement selon 1c) est supprimée.

FIG.1.

FIG.2.

FIG.3.